# NEUE EUROPÄISCHE PATENTSCHRIFT

(11) **EP 0 493 495 B2**
(45) Veröffentlichungstag und Bekanntmachung des Hinweises auf die Entscheidung über den Einspruch: **03.04.2002**
(45) Hinweis auf die Patenterteilung: 19.10.1994
(21) Anmeldenummer: 90914721.7
(22) Anmeldetag: 19.09.1990
(51) Int. Cl.: C07H 15/04, B01D 1/26

(54) **VERFAHREN ZUM DESTILLATIVEN ABTRENNEN VON ALKOHOLEN**
METHOD FOR DISTILLATIVE SEPARATION OF ALCOHOLS
PROCEDE POUR LA SEPARATION D'ALCOOLS PAR DISTILLATION

(30) Priorität: 27.09.1989 DE 3932173
(43) Veröffentlichungstag der Anmeldung: 08.07.1992
(73) Patentinhaber: Cognis Deutschland GmbH, 40589 Düsseldorf (DE)
(72) Erfinder: JOHANNISBAUER, Wilhelm, D-4006 Erkrath 1 (DE); KÖRNER, Hermann, D-4000 Düsseldorf (DE); NITSCHE, Michael, D-5650 Solingen (DE)
(86) Internationale Anmeldenummer: EP9001582
(87) Internationale Veröffentlichungsnummer: WO9104980

(56) Entgegenhaltungen:
- EP-A- 0 092 876
- EP-A- 0 362 671
- DE-A- 3 001 064
- DE-A- 3 529 983
- Ullmanns Enzyklopädie der technischen Chemie, 4.Auflage (1972), Band 2, Seiten 650-663
- Winnacker-Küchler, Chemische Technologie (1984), Band 1, Seiten 364-366

## Beschreibung

Die Erfindung betrifft ein Verfahren zum destillativen Abtrennen von Alkoholen mit Kettenlängen bis 30, insbesondere zwischen 8 und 18 Kohlenstoffatomen, aus einem Gemisch von Alkylglycosiden und bei der Herstellung dieser Alkylglycoside nicht umgesetzten Alkoholen, wobei die Alkohole in zwei Stufen abgetrennt werden.

Unter Alkylglycosiden sind Reaktionsprodukte aus beliebigen Zuckerarten und aliphatischen Alkoholen zu verstehen. Als Zucker kommen Monosaccharide, wie Pentosen und Hexosen, Disaccharide, wie Saccharose und Maltose, und Polysaccharide wie Stärke, in Betracht. Diese Kohlenhydrate werden mit gesättigten oder ungesättigten aliphatischen Alkoholen mit Kettenlängen von 1 bis 30 Kohlenstoffatomen, insbesondere 8 bis 18 Kohlenstoffatomen in Gegenwart eines geeigneten sauren Katalysators umgesetzt. Dabei erhält man Gemische aus Alkylmonoglycosiden und Alkylpoly- oder Alkyloligoglycosiden die nachstehend als Alkylglycoside bezeichnet werden. Die Reaktion wird in der Regel mit einem großen Überschuß an Alkohol durchgeführt, so daß man als Reaktionsprodukt ein Gemisch aus Alkylglycosiden und Alkoholen erhält. Da die Alkohole die anwendungstechnischen Eigenschaften der Alkylglycodside beeinträchtigen, müssen sie von dem Alkylglycosiden abgetrennt werden. Anschließend empfiehlt sich aus wirtschaftlichen Gründen eine Rückführung der abgetrennten Alkohole in den Reaktor.

Da die Siedepunkte der Alkohole insbesondere bei Verwendung von Fettalkohlen, also von Alkoholen mit 8 oder mehr Kohlenstoffatomen, die vornehmlich aus technischen Fetten gewonnen werden, sehr hoch liegen und da sich bereits bei Temperaturen über 150 °C nicht umgesetzte Zukkerreste unter unerwünschter Dunkelfärbung zersetzen, wird die destillative Abtrennung entweder unter Zugabe von Schleppmitteln oder im Feinvakuum (EP-PS 92876) durchgeführt. Die Zugabe von Schleppmitteln weist den Nachteil auf, daß das Produkt mit zusätzlichen Stoffen in Kontakt kommt, die einerseits die Produktqualität mindern können und die andererseits einen vermehrten apparativen Aufwand für die Abtrennung und Rückführung des Schleppmittels bedingen. Bei Destillation ohne Verwendung von Schleppmitteln kommen aufgrund der Temperaturempfindlichkeit und aufgrund der erhöhten Viskosität der vom Alkohol befreiten Alkylglycoside Dünnschichtverdampfer Zum Einsatz. Dünnschichtverdampfer sind Apparate, in denen das Einsatzgemisch auf eine beheizte Wand aufgegeben wird, wo es durch, in der Regel rotierende, Wischelemente mechanisch verteilt wird, so daß ein sehr guter Wärmeaustausch zwischen Wand und Fluid erreicht wird und lokale Überhitzungen vermieden werden.

In der europäischen Patentschrift 92876 wird die einstufige Destillation des Rohprodukts in einem Dünnschichtverdampfer vorgeschlagen. Sofern der Alkoholgehalt im Alkylglycosid auf Werte unter 1 Gew.-% gesenkt werden muß, und dies ist zur Erzielung eines in Wasser klar löslichen Produkts meist erforderlich, ergibt sich eine sehr geringe mittlere Heizflächenbelastung und damit die Notwendigkeit zum Einsatz eines sehr großen Verdampferapparates, der das Verfahren unwirtschaftlich werden läßt.

In der früheren, nachveröffentlichten deutschen Patentanmeldung P 38 33 780.0 wird ein Verfahren der eingangs genannten Art, und zwar die zweistufige Destillation des Rohprodukts in einer Anlagenkombination aus Dünnschichtverdampfer und Kurzwegverdampfer vorgeschlagen. Als Kurzwegverdampfer werden Dünnschichtverdampfer mit innenliegendem Kondensator bezeichnet, die bei Verdampferdrucken zwischen 10⁻¹ und 10⁻⁴ mbar betrieben werden. Durch die geringe Distanz zwischen Heiz- und Kondensationsfläche kommt es bei Auftreten von Siedeverzügen zum Mitreißen der schwerer flüchtigen Komponente, so daß diese vermehrt ins Destillat gelangt. Das Mitreißen des Produkts zieht sowohl eine Reduzierung der Ausbeute als auch eine Qualitätsverminderung des Destillats nach sich, wobei das mit Produkt verunreinigte Destillat nicht ohne zusätzliche Reinigung in den Reaktionsteil zurückgeführt werden kann.

Als weiterer Stand der Technik werden genannt: EP-PS 32 252, die nachveröffentlichte Patentanmeldung P 38 27 534.1, DE-OS 37 23 826 A1.

Die Aufgabe der Erfindung bestand in der Entwicklung eines Verfahrens zur destillativen Trennung des Gemischs aus Alkylglycosid und Alkohol, das bei vertretbaren Gesamtkosten den Alkoholgehalt im Produkt auf beliebig wählbare Werte zwischen 0,1 und 10 Gew.-% zu senken vermag und gleichzeitig ein hochwertiges, d.h. nahezu alkylglycosidfreies Destillat liefert.

Diese Aufgabe wird bei einem Verfahren der eingangs genannten Art erfindungsgemäß dadurch gelöst, daß in der ersten Stufe ein Fallfilmverdampfer bei einem Betriebsdruck von 3 bis 20 mbar und in der zweiten Stufe ein Dünnschichtverdampfer eingesetzt wird.

Insbesondere wird eine Sumpftemperatur des Fallfilmverdampfers von 100 bis 220 °C, insbesondere 140 bis 180 °C vorgeschlagen. Ein Betriebsdruck des Fallfilmverdampfers von insbesondere 3 bis 10 mbar ist außerdem vorteilhaft.

Beim Dünnschichtverdampfer ist eine Sumpftemperatur von 120 bis 250 °C, insbesondere 160 bis 230 °C günstig. Der Betriebsdruck des Dünnschichtverdampfers sollte bei Werten von 0,1 bis 5 mbar, insbesondere 0,5 bis 1,5 mbar liegen.

Als vorteilhaft hat es sich ferner herausgestellt, wenn die Betriebsparameter des Fallfilmverdampfers so eingestellt werden, daß an dessem Auslaß Alkoholgehalte von 10 bis 50 Gew.-%, insbesondere 20 bis 30 Gew.-% erhalten werden.

Ferner ist es günstig, wenn der Fallfilmverdampfer mit äußerem Zwangsumlauf betrieben wird.

Schließlich wird vorgeschlagen, daß die Betriebsparameter des Dünnschichtverdampfers so eingestellt werden, daß an dessem Auslaß Alkoholgehalte von 0,1 bis 10 Gew.-%, insbesondere 0,3 bis 3 Gew.-% erhalten werden.

Durch die Beschränkung der minimalen Alkoholkonzentration im Sumpf der ersten Stufe auf 10 Gew.-% bleibt die Viskosität des Gemisches so niedrig, daß die Verwendung eines gegenüber Dünnschichtverdampfern wesentlich kostengünstigeren Fallfilmverdampfers möglich wird. Um eine ausreichende Umfangsbelastung zu erzielen und somit vollständige Benetzung der Verdampferfläche zu gewährleisten, empfiehlt es sich, den Verdampfer mit äußerem Zwangsumlauf zu betrieben. Bei entsprechender Wahl der Apparategeometrie ist jedoch auch ein Betrieb mit einmaligem Durchlauf ohne weiteres möglich.

Der Einsatz eines Dünnschichtverdampfers als zweite Destillationsstufe vermeidet weitgehend das Mitreißen von Alkylglycosid, so daß im Destillat deutlich weniger als 1 Gew.-% Alkylglycosid gegenüber bis zu 30 Gew.-% Alkylglycosid im Destillat eines Kurzwegverdampfers gefunden werden. Das im Vergleich zum Kurzwegverdampfer schlechtere Vakuum wird durch eine geringfügige Erhöhung der Heizmitteltemperatur kompensiert. Die erhöhte thermische Belastung des Produkts führt zu geringfügig schlechterer Farbqualität des unbehandelten Sumpfproduktes. Nach der in der Regel abschließend durchgeführten chemischen Bleichung des in Wasser gelösten Produktes sind jedoch keine Unterschiede in der Farbqualität mehr festzustellen.

Nachfolgend wird ein Ausführungsbeispiel der Erfindung beschrieben.

In einer zweistufigen Destillationsanlage, bestehend aus einem Fallfilmverdampfer mit 4,5 m² Verdampferfläche und einem Dünnschichtverdampfer mit 1 m² Verdampferfläche, wurde ein Gemisch aus Alkylglucosid und Fettalkoholen getrennt. Das Alkylglucosid war aus Kartoffelstärke und einem Gemisch aus Laurin- und Myristinalkhol synthetisiert worden, wobei das Molverhältnis Glykose zu Alkohol 1:4,5 betragen hatte. Der Fallfilmverdampfer wurde mit einer Heizmitteltemperatur von 180 °C, einer Sumpftemperatur von 160 °C und einem Druck von ca. 8 mbar betrieben. Der Dünnschichtverdampfer wurde mit einer Heizmitteltemperatur von 210 °C, einer Sumpftemperatur von 200 °C und einem Druck von ca. 1 mbar betrieben. Bei stationärem Betriebszustand wurde dem Fallfilmverdampfer ein Massenstrom von 300 kg/h Reaktionsgemisch mit einem Fettalkohlgehalt von ca. 70 Gew.-% zugeführt. In der ersten Destillationsstufe wurde der Fettalkoholgehalt auf ca. 30 Gew.-% reduziert, so daß dem Dünnschichtverdampfer ein Massenstrom von ca. 130 kg/h zugeführt werden konnte. Das Sumpfprodukt der zweiten Destillationsstufe enthielt ca. 0,8 Gew.-% Rest-Fettalkohol.

## Patentansprüche

1. Verfahren zum destillativen Abtrennen von Alkoholen mit Kettenlängen bis 30, insbesondere zwischen 8 und 18 Kohlenstoffatomen, aus einem Gemisch von Alkylglykosiden und bei der Herstellung dieser Alkyglycoside nicht umgesetzten Alkoholen, wobei die Alkohole in zwei Stufen abgetrennt werden, **dadurch gekennzeichnet, daß** in der ersten Stufe ein Fallfilmverdampfer bei einem Betriebsdruck von 3 bis 20 mbar und in der zweiten Stufe ein Dünnschichtverdampfer eingesetzt wird.

2. Verfahren nach Anspruch 1, **gekennzeichnet durch** eine Sumpftemperatur des Fallfilmverdampfers von 100 bis 220 °C, insbesondere 140 bis 180 °C.

3. Verfahren nach Anspruch 1, **gekennzeichnet durch** einen Betriebsdruck des Fallfilmverdampfers von 3 bis 10 mbar.

4. Verfahren nach Anspruch 1, **gekennzeichnet durch** eine Sumpftemperatur des Dünnschichtverdampfers von 120 bis 250 °C, insbesondere 160 bis 230 °C.

5. Verfahren nach Anspruch 1, **gekennzeichnet durch** einen Betriebsdruck des Dünnschichtverdampfers von 0,1 bis 5 mbar, insbesondere 0,5 bis 1,5 mbar.

6. Verfahren nach Anspruch 1, **dadurch gekennzeichnet, daß** die Betriebsparameter des Fallfilmverdampfers so eingestellt werden, daß an dessen Auslaß Alkoholgehalte von 10 bis 50 Gew.-%, insbesondere 20 bis 30 Gew.-% erhalten werden.

7. Verfahren nach Anspruch 1, **dadurch gekennzeichnet, daß** der Fallfilmverdampfer mit äußerem Zwangsumlauf betrieben wird.

8. Verfahren nach Anspruch 1, **dadurch gekennzeichnet, daß** die Betriebsparameter des Dünnschichtverdampfers so eingestellt werden, daß an dessen Auslaß Alkoholgehalte von 0,1 bis 10 Gew.-%, insbesondere 0,3 bis 3 Gew.-% erhalten werden.

## Claims

1. A process for the removal of alcohols containing up to 30 and, more particularly, between 8 and 18 carbon atoms by distillation from a mixture of alkyl glycosides and alcohols remaining unreacted in the production of these alkyl glycosides, the alcohols being removed in two stages, **characterized in that** a falling-film evaporator with an operating temperature of 3 to 20 mbar is used in the first stage while a thin-layer evaporator is used in the second stage.

2. A process as claimed in claim 1, **characterized by** a bottom temperature of the falling-film evaporator of 100 to 220°C and, more particularly, 140 to 180°C.

3. A process as claimed in claim 1, **characterized by** an operating pressure of the falling film evaporator of 3 to 10 mbar.

4. A process as claimed in claim 1, **characterized by** a bottom temperature of the thin-layer evaporator of 120 to 250°C and, more particularly, 160 to 230°C.

5. A process as claimed in claim 1, **characterized by** an operating pressure of the thin-layer evaporator of 0.1 to 5 mbar and, more particularly, 0.5 to 1.5 mbar.

6. A process as claimed in claim 1, **characterized in that** the operating parameters of the falling film evaporator are adjusted in such a way that alcohol contents of 10 to 50% by weight and, more particularly, 20 to 30% by weight are obtained at its outlet.

7. A process as claimed in claim 1, **characterized in that** the falling-film evaporator is operated with external forced circulation.

8. A process as claimed in claim 1, **characterized in that** the operating parameters of the thin-layer evaporator are adjusted in such a way that alcohol contents of 0.1 to 10% by weight and, more particularly, 0.3 to 3% by weight are obtained at its outlet.

## Revendications

1. Procédé de séparation par distillation d'alcools possédant une longueur de chaîne allant jusqu'à 30, en particulier comprise entre 8 et 18 atomes de carbone, à partir d'un mélange d'alkylglycosides et d'alcools qui ne sont pas entrés en réaction lors de la fabrication de ces alkylglycosides, dans lequel les alcools sont séparés en deux étapes,
**caractérisé en ce que**
l'on met en oeuvre dans la première étape, un évaporateur à ruissellement à une pression de service de 3 à 20 mbars et dans la seconde étape, un évaporateur à couche mince,

2. Procédé selon la revendication 1,
**caractérisé par**
une température de résidu de l'évaporateur à ruissellement de 100 à 220°C, en particulier, de 140 à 180°C.

3. Procédé selon la revendication 1,
**caractérisé par**
une pression de service de l'évaporateur à ruissellement de 3 à 10 mbars.

4. Procédé selon la revendication 1,
**caractérisé par**
une température de résidu de l'évaporateur à couche mince de 120 à 250°C, en particulier, de 160 à 230°C.

5. Procédé selon la revendication 1,
**caractérisé par**
une pression de service de l'évaporateur à couche mince de 0,1 à 5 mbars, en particulier de 0,5 à 1,5 mbar.

6. Procédé selon la revendication 1,
**caractérisé en ce que**
les paramètres de service de l'évaporateur à ruissellement sont réglés de manière telle que l'on obtient à sa sortie des teneurs en alcools de 10 à 50% en poids, en particulier de 20 à 30% en poids.

7. Procédé selon la revendication 1,
**caractérisé en ce que**
l'évaporateur à ruissellement est mis en fonctionnement avec une circulation forcée externe.

8. Procédé selon la revendication 1,
**caractérisé en ce que**
les paramètres de service de l'évaporateur à couche mince sont réglés de manière telle que l'on obtient à sa sortie, des teneurs en alcools de 0,1 à 10% en poids, en particulier de 0,3 à 3% en poids.
